# EUROPEAN PATENT APPLICATION

(11) **EP 4 851 196 A1**
(43) Date of publication of application: **09.12.2026**
(21) Application number: 25748020.2
(22) Date of filing: 27.01.2025
(51) Int. Cl.: C07K 16/46, C07K 16/28

(54) **FUSION ANTIBODY, AND PREPARATION AND USE THEREOF**

(30) Priority: 04.02.2024 CN 202410156442
(71) Applicant: Yichen Therapeutics Limited, Wan Chai, Hong Kong (HK)
(72) Inventor: WANG, Feng, Nantong, Jiangsu 226002 (CN); ZHANG, Yuhan, Nantong, Jiangsu 226002 (CN)
(74) Representative: Studio Torta S.p.A.
(86) International application number: PCT/CN2025/075552
(87) International publication number: WO 2025/162458

(57) **Abstract**

The present invention provides a fusion antibody, which comprises a first polypeptide chain consisting of VH, CH1, hinge and a first Fc, and a second polypeptide chain consisting of VL, CL, hinge and a second Fc. One or more cysteine residues (Cys) on the hinge of the first polypeptide chain and the second polypeptide chain are deleted or mutated to other amino acids, and the cysteine residue at position 214 of CL of the second polypeptide chain is deleted (C214del).

## Description

### Technical Field

The present invention belongs to the field of biopharmaceuticals, and specifically relates to a fusion antibody, preparation and application thereof.

### Background Art

Disulfide bonds are crucial for maintaining the structure and function of antibodies and protein drugs. Therefore, accurate analysis of the disulfide bonds of antibodies and recombinant protein drugs is necessary during their production. For antibodies, mismatched disulfide bonds affect protein structure and function, and even induce antibody aggregation. Disulfide bond mismatch may occur at any stage of cell fermentation or purification processes. Therefore, accurate analysis of disulfide bonds of antibodies and proteins helps researchers timely identify issues during the manufacturing process and adjust process parameters in a timely manner to ensure the quality of the final product.

Under alkaline conditions, disulfide bonds can be decomposed via a β-elimination mechanism to form dehydroalanine and persulfide, which can be further reduced back to cysteine residues. The disulfide bond between the light chain and the heavy chain in IgG molecules is degraded through the beta-elimination mechanism, and the subsequently generated cysteine and dehydroalanine cross-link to form a non-reducible thioether bond. Such thioether bonds account for about 0.4% in recombinant monoclonal IgG1 stored at 4 C, and up to 13.6% in heat-stressed samples (MAbs. 2012 Jan 1;4(1):17-23).

Thioether bond formation in antibodies is observed both in ex vivo storage and in vivo therapy. After intravenous administration of IgG1κ therapeutic antibody, the conversion rate of thioether bonds is approximately 0.1% per day, similar to the ex vivo conversion rate of 0.09% per day. IgG1λ antibodies show a higher conversion rate (0.16% per day) than IgG1κ. In therapeutic or native IgG1 antibodies, thioether bond formation mainly occurs at C214 of LC (EU numbering) and C220 of HC (EU numbering) (J Biol Chem. 2013 Jun 7;288(23):16371-16382).

Furthermore, studies have found that thioether products are observed in the expressed and purified products of certain artificially designed and prepared bispecific antibodies or antibody fusion proteins in mammalian cells. During large-scale production, removal of thioether products requires additional development of corresponding separation and purification methods, thereby further increasing production costs.

Therefore, developing a method that eliminates thioether bond formation (and thus avoids immunogenicity) in bispecific antibodies or antibody fusion proteins, while retaining their stability and efficacy, is of great significance for the production, storage and in vivo therapy of such drugs.

### Summary of the Invention

To address the issue of batch-to-batch quality heterogeneity caused by thioether bond formation in antibody fusion proteins during production and storage, as well as the efficacy and safety risks (e.g., greater immunogenic potential) associated with thioether bond formation during in vivo therapy, the present invention provides a fusion antibody. By modifying cysteine residues (Cys) at specific sites on the hinges of the two polypeptide chains constituting the fusion antibody, no inter-chain thioether product is observed after expression.

Accordingly, in a first aspect, the present invention provides a fusion antibody comprising:
(a) a first polypeptide chain comprising, from N-terminus to C-terminus, antibody heavy chain variable region (VH), antibody heavy chain first constant region (CH1), hinge region (hinge) and first Fc;
(b) a second polypeptide chain comprising, from N-terminus to C-terminus, antibody light chain variable region (VL), antibody light chain constant region (CL), hinge region (hinge) and second Fc; or
   (a) a first polypeptide chain comprising, from N-terminus to C-terminus, VL, CH1, hinge and first Fc;
   (b) a second polypeptide chain comprising, from N-terminus to C-terminus, VH, CL, hinge and second Fc;
   wherein, VH and VL form a first antigen-binding domain, and the cysteine (Cys) at position 214 of CL of the second polypeptide chain is deleted (C214del, EU numbering, used throughout), or mutated to serine (Ser) (C214S), glycine (Gly) (C214G) or threonine (Thr) (C214T).

In some embodiments, the hinges of the first polypeptide chain and the second polypeptide chain are different.

In some embodiments, the hinge of the first polypeptide chain and the hinge of the second polypeptide chain lack the Cys at position 220, i.e., C220del.

In some embodiments, the first polypeptide chain has a hinge as shown in EPKSDKTHTCPPCP (SEQ ID NO:82), and the second polypeptide chain has a hinge as shown in DKTHTCPPCP (SEQ ID NO:101).

In some embodiments, the hinge of the first polypeptide chain and the hinge of the second polypeptide chain are identical.

In some embodiments, the hinge of the first polypeptide chain and the hinge of the second polypeptide chain are derived from IgG1, IgG2, IgG3 or IgG4.

In some embodiments, the hinge of the first polypeptide chain and the hinge of the second polypeptide chain are derived from IgG1.

In some embodiments, the IgG1-derived hinge of the first polypeptide chain and the second polypeptide chain is EPKSCDKTHTCPPCP (E216-P230) (SEQ ID NO:81).

In some embodiments, one or more Cys on the hinges of the first polypeptide chain and the second polypeptide chain are deleted or mutated to other amino acids.

In some embodiments, the first Cys on the IgG1-derived hinges of the first polypeptide chain and the second polypeptide chain is deleted (C220del).

In some embodiments, the first Cys on the IgG1-derived hinges of the first polypeptide chain and the second polypeptide chain is mutated to other amino acids; preferably, the first Cys is mutated to Ser (C220S), Gly (C220G) or Thr (C220T).

In some embodiments, when the first Cys on the hinges of the IgG1-derived first polypeptide chain and second polypeptide chain is deleted (C220del), the second Cys on the hinges of the first and second polypeptide chains is deleted (C226del), or mutated to Ser (C226S), Gly (C226G), or Thr (C226T).

In some embodiments, when the first Cys on the hinges of the IgG1-derived first and second polypeptide chain is mutated to Ser (C220S), Gly (C220G), or Thr (C220T), the second Cys on the hinges of the first and second polypeptide chains is deleted (C226del), or mutated to Ser (C226S), Gly (C226G), or Thr (C226T).

In some embodiments, when the first Cys on the hinges of the IgG1-derived first and second polypeptide chain is deleted (C220del), the third Cys on the hinges of the first and second polypeptide chains is deleted (C229del), or mutated to Ser (C229S), Gly (C229G), or Thr (C229T).

In some embodiments, when the first Cys on the hinges of the IgG1-derived first and second polypeptide chain is mutated to Ser (C220S), Gly (C220G), or Thr (C220T), the third Cys on the hinges of the first and second polypeptide chains is deleted (C229del), or mutated to Ser (C229S), Gly (C229G), or Thr (C229T).

In some embodiments, when the first Cys (C220del) and the second Cys (C226del) on the IgG1-derived hinges of the first and second polypeptide chains are deleted, the third Cys on the hinges on the hinges of the first and second polypeptide chains is deleted (C229del), or mutated to Ser (C229S), Gly (C229G), or Thr (C229T).

In some embodiments, when the first Cys on the hinges of the IgG1-derived first and second polypeptide chain is mutated to Ser (C220S), Gly (C220G), or Thr (C220T) and the second Cys is deleted (C226del), the third Cys on the hinges of the first polypeptide chain and the second polypeptide chain is deleted (C229del), or mutated to Ser (C229S), Gly (C229G), or Thr (C229T).

In some embodiments, when the first Cys on the hinges of the IgG1-derived first and second polypeptide chain is deleted (C220del) and the second Cys is mutated to Ser (C226S), Gly (C226G), or Thr (C226T), the third Cys on the hinges of the first polypeptide chain and the second polypeptide chain is deleted (C229del), or mutated to Ser (C229S), Gly (C229G), or Thr (C229T).

In some embodiments, when the first Cys on the hinges of the IgG1-derived first and second polypeptide chain is mutated to Ser (C220S), Gly (C220G), or Thr (C220T) and the second Cys is mutated to Ser (C226S), Gly (C226G), or Thr (C226T), the third Cys on the hinges of the first polypeptide chain and the second polypeptide chain is deleted (C229del), or mutated to Ser (C229S), Gly (C229G), or Thr (C229T).

In some embodiments, the hinges of the first polypeptide chain and the second polypeptide chain are derived from IgG4.

In some embodiments, the hinge of the IgG4-derived first and second polypeptide chain is ESKYGPPCPSCP (E216-P230) (SEQ ID NO:94).

In some embodiments, the Cys at position 200 on the CH1 of the first polypeptide chain is deleted (C200del).

In some embodiments, the Cys at position 200 on the CH1 of the first polypeptide chain is mutated to another non-thiol-containing amino acid; preferably, mutated to Ser (C200S), Gly (C200G), or Thr (C200T).

In some embodiments, the hinges of IgG2-derived first and second polypeptide chains have the amino acid sequence as shown in ERKCCVECPPCP (E216-P230) (SEQ ID NO:100).

In some embodiments, the first Fc and the second Fc are derived from IgG1, IgG2, IgG3, or IgG4.

In some embodiments, the first Fc and the second Fc comprise a modified CH3 domain, wherein the modified CH3 domain comprises amino acid substitutions that promote heterodimerization of the first Fc and the second Fc.

In some embodiments, one of the first Fc and the second Fc comprises the amino acid substitution T366W, and the other of the first Fc and the second Fc comprises the amino acid substitutions T366S, L368A, and Y407V; preferably, the one of the first Fc and the second Fc comprising the amino acid substitution T366W further comprises the amino acid substitution S354C, and the other of the first Fc and the second Fc comprising the amino acid substitutions T366S, L368A, and Y407V further comprises the amino acid substitution Y349C.

In some embodiments, one of the first Fc and the second Fc comprises the amino acid substitutions E356K and R409K, and the other of the first Fc and the second Fc comprises the amino acid substitutions R409K and K439E.

In some embodiments, the N-terminus of one of the first polypeptide chain and the second polypeptide chain further comprises VH2, and the N-terminus of the other of the first polypeptide chain and the second polypeptide chain further comprises VL2, wherein VH2 and VL2 form a second antigen-binding domain.

In some embodiments, the first binding domain of the fusion antibody binds to EGFR antigen; in some embodiments, the fusion antibody further comprises a second binding domain that binds to cMET antigen.

In some embodiments, the fusion antibody of the present invention comprises first and second polypeptide chains having amino acid sequences selected from the following pairs: SEQ ID NO:1 and SEQ ID NO:2; SEQ ID NO:3 and SEQ ID NO:4; SEQ ID NO:5 and SEQ ID NO:6; SEQ ID NO:7 and SEQ ID NO:8; SEQ ID NO:9 and SEQ ID NO:10; SEQ ID NO:11 and SEQ ID NO:12; SEQ ID NO:13 and SEQ ID NO:14; SEQ ID NO:15 and SEQ ID NO:16; SEQ ID NO:17 and SEQ ID NO:18; SEQ ID NO:19 and SEQ ID NO:20; SEQ ID NO:21 and SEQ ID NO:22; SEQ ID NO:23 and SEQ ID NO:24; SEQ ID NO:25 and SEQ ID NO:26; SEQ ID NO:27 and SEQ ID NO:28; SEQ ID NO:29 and SEQ ID NO:30; SEQ ID NO:31 and SEQ ID NO:32; SEQ ID NO:33 and SEQ ID NO:34; SEQ ID NO:35 and SEQ ID NO:36; SEQ ID NO:37 and SEQ ID NO:38; SEQ ID NO:39 and SEQ ID NO:40; SEQ ID NO:41 and SEQ ID NO:42; SEQ ID NO:43 and SEQ ID NO:44; SEQ ID NO:45 and SEQ ID NO:46; SEQ ID NO:47 and SEQ ID NO:48; SEQ ID NO:49 and SEQ ID NO:50; SEQ ID NO:51 and SEQ ID NO:52; SEQ ID NO:53 and SEQ ID NO:54; SEQ ID NO:55 and SEQ ID NO:56; SEQ ID NO:57 and SEQ ID NO:58; SEQ ID NO:59 and SEQ ID NO:60; SEQ ID NO:61 and SEQ ID NO:62; SEQ ID NO:63 and SEQ ID NO:64; SEQ ID NO:65 and SEQ ID NO:66; SEQ ID NO:67 and SEQ ID NO:68; SEQ ID NO:69 and SEQ ID NO:70; SEQ ID NO:71 and SEQ ID NO:72; SEQ ID NO:77 and SEQ ID NO:78; SEQ ID NO:79 and SEQ ID NO:80; SEQ ID NO:102 and SEQ ID NO:103; SEQ ID NO:104 and SEQ ID NO:105; SEQ ID NO:106 and SEQ ID NO:107; SEQ ID NO:107 and SEQ ID NO:108; SEQ ID NO:109 and SEQ ID NO:110; SEQ ID NO:111 and SEQ ID NO:112.

A second aspect of the present invention provides a fusion antibody conjugate, consisting of a fusion antibody and a cytotoxic drug, wherein the fusion antibody consists of two polypeptide chains:
(a) a first polypeptide chain, comprising from N-terminus to C-terminus a VH, a CH1, a hinge, and a first Fc; and
(b) a second polypeptide chain, comprising from N-terminus to C-terminus a VL, a CL, a hinge, and a second Fc;
or,
(a) a first polypeptide chain, comprising from N-terminus to C-terminus a VL, a CH1, a hinge, and a first Fc; and
(b) a second polypeptide chain, comprising from N-terminus to C-terminus a VH, a CL, a hinge, and a second Fc;
wherein, the Cys at position 214 of the CL of the second polypeptide chain is deleted (C214del, EU numbering, used throughout), or mutated to Ser (C214S), Gly (C214G), or Thr (C214T); and the cytotoxic drug is conjugated to Cys on the hinges of the first and the second polypeptide chain.

In some embodiments, the cytotoxic drug is conjugated to the Cys at position 226 and/or position 229 on the hinges of the first and the second polypeptide chain.

A third aspect of the present invention provides a polynucleotide encoding the aforementioned fusion antibody.

A fourth aspect of the present invention provides a vector comprising the polynucleotide encoding the fusion antibody.

A fifth aspect of the present invention provides a host cell comprising the vector containing the polynucleotide encoding the aforementioned fusion antibody.

A final aspect of the present invention provides a pharmaceutical composition comprising the aforementioned fusion antibody.

The present invention finds that when the last Cys at the end of the CL of the second polypeptide chain is deleted (C214del) or mutated to Serine (Ser) (C214S), and the hinge of the first and the second polypeptide chain lack the Cys at position 220 (C220del) or the Cys at position 220 is mutated to Ser (C220S), the formation of non-target bands (thioether products) that cannot be reduced under DTT reducing conditions can be effectively reduced for the fusion antibody. On the foregoing basis, further deletion (C226del) or mutation to Ser (C226S) of the second Cys and/or deletion (C229del) or mutation to Ser (C229S) of the third Cys in the hinge of the first polypeptide chain and the hinge of the second polypeptide chain still effectively prevents the formation of non-target bands (thioether products).

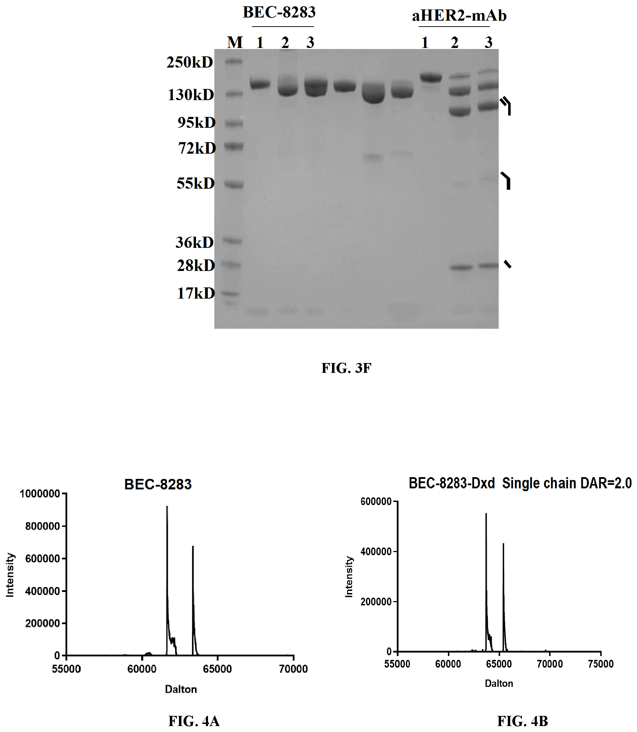

The applicant further finds that after the disulfide bonds on the hinge of the fusion antibody of the present invention are opened with the reducing agent TCEP (Tris(2-carboxyethyl)phosphine hydrochloride), cytotoxic drugs such as MMAE, Dxd, or DUPA can be conjugated to the thiol groups generated by reduction of Cys on the hinge, and the resulting conjugate exhibits stable structure (see the bands between 130-250kD for BEC8283 in Figure 3F). In contrast, conjugates obtained by other ADC conjugation techniques currently on the market show multiple bands on non-reducing SDS-PAGE (see lanes 2-3 for aHER2 mAb in Figure 3F), indicating that the fusion antibody-based conjugate of the present invention has better structural stability. Moreover, since all Cys involved in conjugation are located in the hinge region, the resulting conjugate has a more uniform DAR value, which is beneficial for ensuring consistency in clinical efficacy and safety.

### Brief Description of the Drawings

The accompanying drawings, which constitute a part of this application, are provided for a further understanding of the present invention. The schematic embodiments and descriptions thereof are used to explain the present invention and do not constitute an improper limitation thereof. In the drawings:
Figure 1 is a schematic structural diagram of the fusion antibody of the present invention.

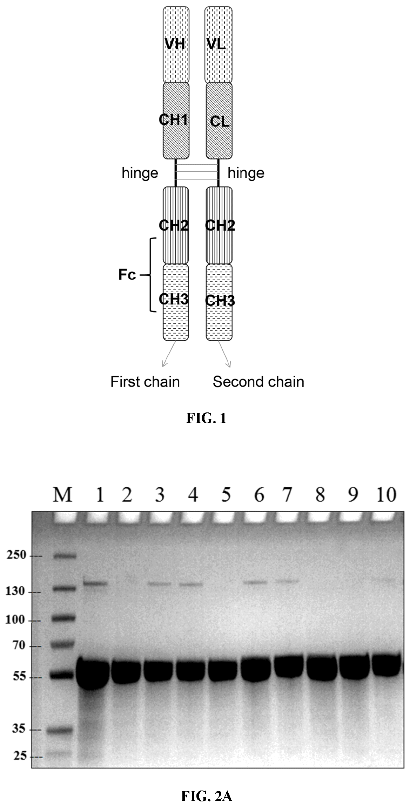

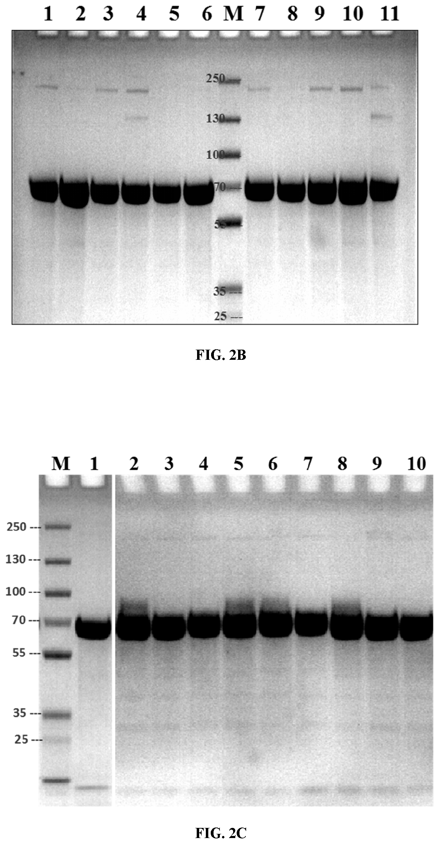

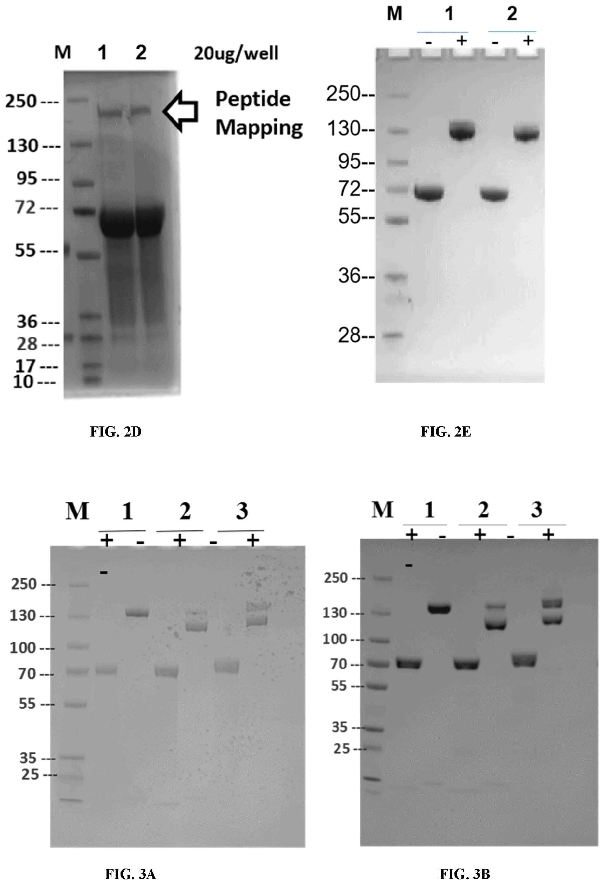

Figures 2A-2C are SDS-PAGE gels images of the fusion antibodies of Example 2 after treatment with the reducing agent DTT. Lanes 1-10 in Figure 2A correspond to MTE6263, MTE6465, MTE6667, MTE6869, MTE7071, MTE7273, MTE7475, MTE7677, MTE7879, MTE8081 respectively; Lanes 1-11 in Figure 2B correspond to BEC2728, BEC8283, BEC2930, BEC3132, BEC3334, BEC3536, BEC3738, BEC3940, BEC4142, BEC4344, BEC4546 respectively; Lanes 1-10 in Figure 2C correspond to BEC9293, BEC9495, BEC9697, BEC9899, BEC0001, BEC0203, BEC0405, BEC0607, BEC0809, BEC1011 respectively; Figure 2D shows an SDS-PAGE gel image under DTT reducing condition, wherein lane 1 is PEC-3132 and lane 2 is PEC-9903; In Figure 2E, "-" indicates no DTT treatment, and "+" indicates DTT treatment, wherein 1 is PEC-1415, and 2 is PEC-1516.
Figures 3A-3F are SDS-PAGE gel images of fusion antibodies and their conjugates from Examples 2 and 3, where "-" and "+" indicate without and with reducing agent DTT, respectively.
Figure 3A shows BEC9293 fusion antibody and its conjugate, wherein 1 is BEC9293 fusion antibody, 2 is BEC9293 treated with Tcep, and 3 is DUPA-conjugated BEC9293 treated with Tcep.
Figure 3B shows BEC9495 fusion antibody and its conjugate, wherein 1 is BEC9495 fusion antibody, 2 is BEC9495 treated with Tcep, and 3 is DUPA-conjugated BEC9495 treated with Tcep.

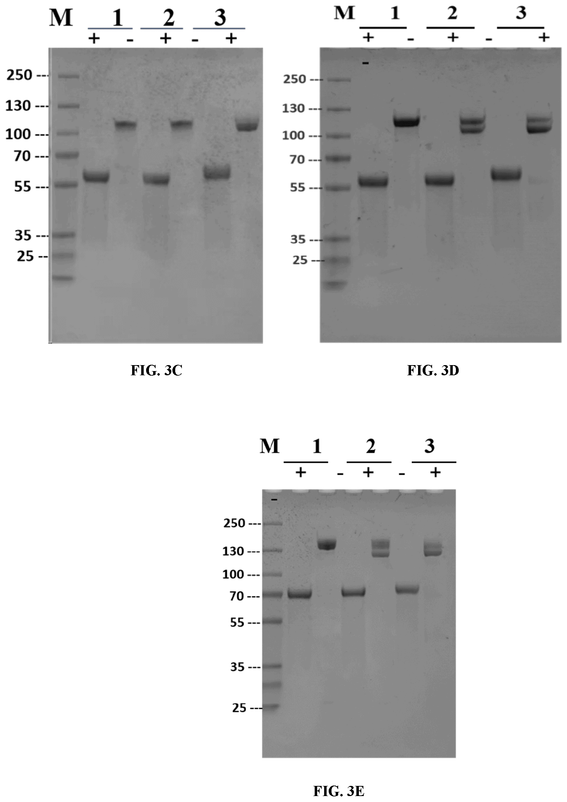

Figure 3C shows MTE6465 fusion antibody and its conjugate, wherein 1 is MTE6465 fusion antibody, 2 is MTE6465 treted with Tcep, and 3 is DUPA-conjugated MTE6465 treated with Tcep.
Figure 3D shows MTE9091 fusion antibody and its conjugate, wherein 1 is MTE9091 fusion antibody, 2 is MTE9091 treated with Tcep, and 3 is DUPA-conjugated MTE9091 treated with Tcep.
Figure 3E shows MTE8687 fusion antibody and its conjugate, wherein 1 is MTE8687 fusion antibody, 2 is MTE8687 treated with Tcep, and 3 is DUPA-conjugated MTE8687 treated with Tcep.
Figure 3F shows BEC8283, aHER2 antibody and their conjugates without DTT reduction, wherein 1 indicates the sample treated with Tcep, 2 indicates Dxd-conjugated BEC8283 treated with Tcep, and 3 indicates MMAE-conjugated BEC8283 treated with Tcep.
Figures 4A-4D show mass spectrometry characterization of fusion antibodies and Dxd-conjugated fusion antibodies.

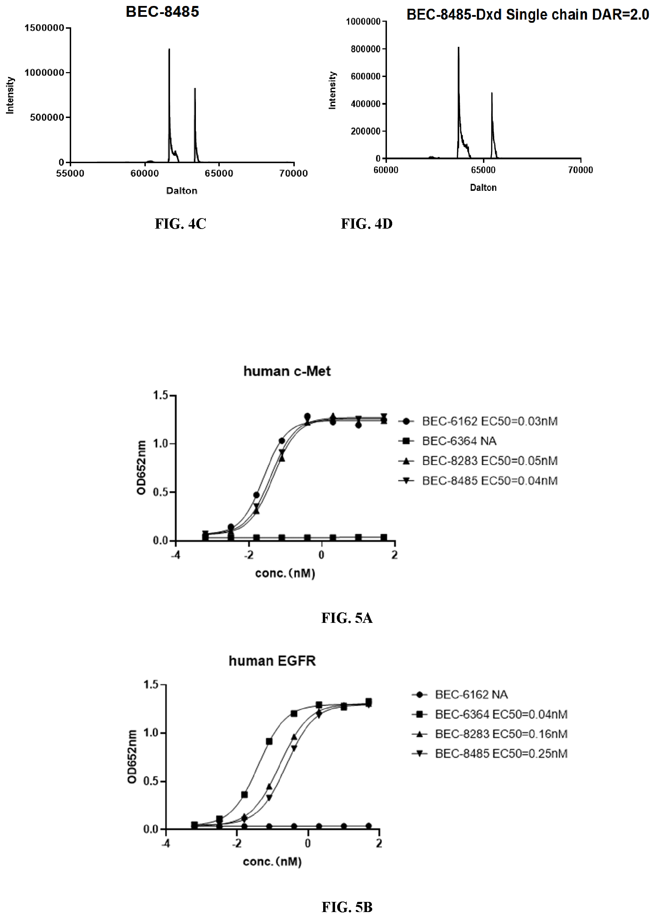

Figures 5A and 5B show the binding activity of the fusion antibodies of Example 3 to antigens c-MET and EGFR, respectively, as detected by ELISA.

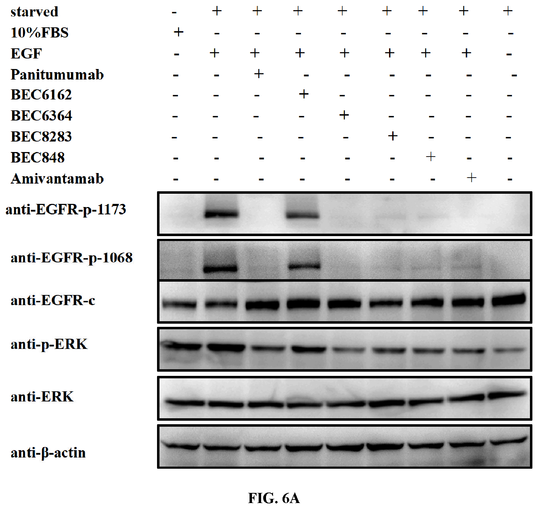

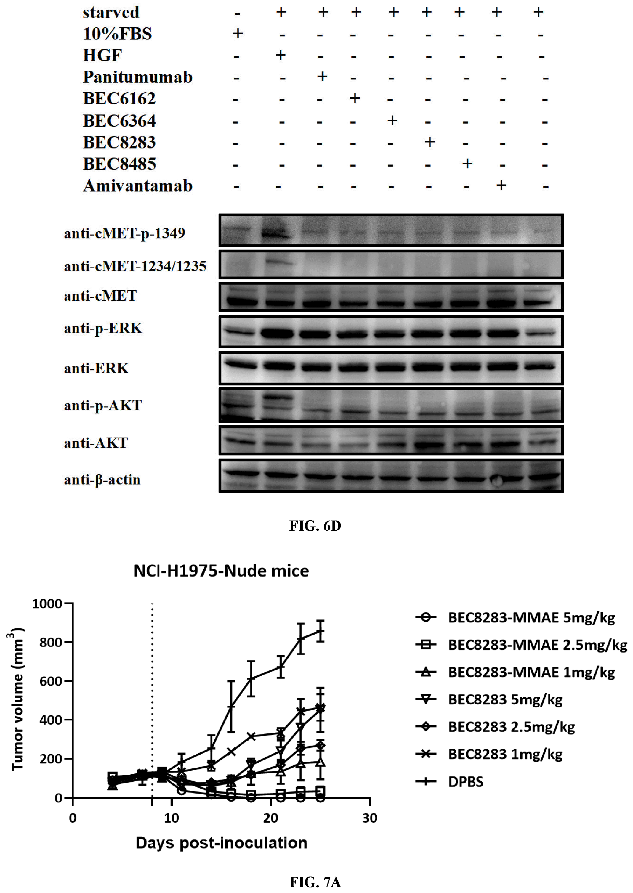

Figures 6A-6D show the inhibition of different cell signaling pathways by the fusion antibodies as detected by Western Blot.

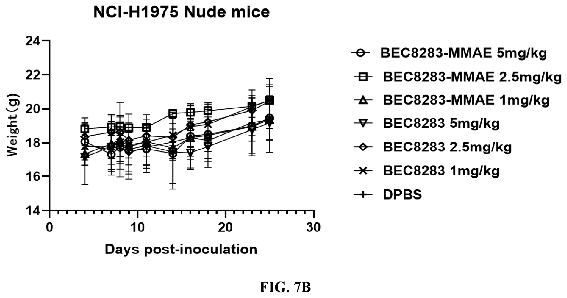

Figure 7A is a graph showing changes in tumor size in tumor-bearing mice after administration of the bispecific antibody and its conjugate, and Figure 7B is a graph showing changes in body weight of the tumor-bearing mice.

### Detailed Description

The present invention is described in detail herein with reference to the following definitions and examples. The contents of all patents and publications mentioned herein, including all sequences disclosed in those patents and publications, are expressly incorporated by reference.

As used herein, "antibody fusion" refers to an antibody or antibody-based fusion protein consisting of two different polypeptide chains each comprising an Fc, wherein the Fc of one polypeptide chain forms an Fc dimer with the Fc of the other polypeptide chain, and the two polypeptide chains form at least one antigen-binding domain.

As used herein, "Fc" is used to define a C-terminal domain of an immunoglobulin heavy chain that contains at least a portion of the constant region. It refers to a polypeptide comprising the constant region of an antibody (excluding the first constant immunoglobulin domain). Thus, Fc refers to the last two constant immunoglobulin domains of human IgA, IgD, and IgG, and the last three constant immunoglobulin domains of IgE and IgM. Due to the homodimeric nature of molecules containing Fc regions, the first Fc and the second Fc of the present invention comprise modified CH3 domains, wherein the modified CH3 domains comprise amino acid mutations that promote heterodimerization of the first Fc and the second Fc (e.g., chimeric mutations, complementary mutations, lock-and-dock mutations, knob-into-hole mutations, charge mutations, strand-exchange engineered domain (SEED) mutations, and the like). Thus, the first Fc and the second Fc may be selected from IgG, IgA, IgE, or IgM isotypes; further, the first Fc and the second Fc may be independently selected from IgG1, IgG2, IgG3, or IgG4. In a specific embodiment, one of the first Fc and the second Fc comprises the amino acid substitution T366W, and the other comprises T366S, L368A, and Y407V; in a specific embodiment, one comprises T366W and S354C, and the other comprises Y349C, T366S, L368A, and Y407V; in a specific embodiment, one comprises E356K and R409K, and the other comprises R409K and K439E; in a specific embodiment, one comprises K409R, and the other comprises L368E; in a specific embodiment, one comprises F405L, and the other comprises K409R; in a specific embodiment, one comprises S364H and F405A, and the other comprises Y349T and T394F; in a specific embodiment, one comprises T350V, L351Y, F405A, and Y407V, and the other comprises T350V, T366L, K392L, and T394W; in a specific embodiment, one comprises K409D and K392D, and the other comprises D399K and E356K; in a specific embodiment, one comprises K360E and K409W, and the other comprises Q347R, D399V, and F405T; in a specific embodiment, one comprises F405L, D356E, and L358M, and the other comprises K409R, D356E, and L358M; in a specific embodiment, one comprises T366S, L368A, Y407V, D356E, and L358M, and the other comprises T366W, D356E, and L358M.

As used herein, "hinge" includes the upper region and lower hinge regions of an antibody, and may be derived from IgA, IgB, IgM, IgE, or IgG. When derived from IgG, it may be selected from IgG1, IgG2, IgG3, or IgG4. When selected from IgG1, its sequence is EPKSCDKTHTCPPCP (E216-P230, EU numbering) (SEQ ID NO:81), wherein: (1) C220 is deleted or mutated to another amino acid that does not contain a sulfhydryl group; (2) C220 is deleted or mutated to another amino acid lacking a sulfhydryl group, and C226 is retained, deleted, or mutated to another amino acid that does not contain a sulfhydryl group; (3) C220 is deleted or mutated to another amino acid that does not contain a sulfhydryl group, C226 is retained, deleted, or mutated to another amino acid that does not contain a sulfhydryl group, and C229 is retained, deleted, or mutated to another amino acid that does not contain a sulfhydryl group; (4) C220 is deleted or mutated to another amino acid that does not contain a sulfhydryl group, and C229 is retained, deleted, or mutated to another amino acid that does not contain a sulfhydryl group. When selected from IgG4, its sequence is ESKYGPPCPPCP (E216-P230) (SEQ ID NO:94).

### Examples

The examples are provided for illustration only and are not intended to limit the present invention in any way.

### Example 1. Vector construction

Gene fragments of VL and VH constituting the antibody and gene fragments for the antibody constant regions were synthesized, respectively. The above gene fragments were amplified by PCR. The amplified VH gene fragment was linked to the antibody constant region gene fragment by overlap PCR to serve as the first chain; the amplified VL gene fragment was linked to the antibody constant region gene fragment to serve as the second chain, as shown in Figure 1. These fragments were further linked to the pFuse vector (InvivoGen, CA) used for eukaryotic expression by homologous recombination, and transformed into competent E. coli DH5α cells. Antibiotic selection was performed on LB plates. After positive clones were selected, plasmids were extracted using an endotoxin-free plasmid extraction kit, and the extracted sequences were verified by sequencing. The nucleotide and amino acid sequences for each construct are as follows:

**Table 1 Fusion antibody sequences and hinges**

| Fusion antibody | Chain | Sequence No. | Hinge |
|---|---|---|---|
| MTE6263 | First chain | SEQ ID NO.1 | hinge1(IgG1 WT) |
| | Second chain | SEQ ID NO.2 | |
| MTE6465 | First chain | SEQ ID NO.3 | hinge2(IgG1 C220del) |
| | Second chain | SEQ ID NO.4 | |
| MTE6667 | First chain | SEQ ID NO.5 | hinge5(IgG1 C226del) |
| | Second chain | SEQ ID NO.6 | |
| MTE6869 | First chain | SEQ ID NO.7 | hinge6(IgG1 C229del) |
| | Second chain | SEQ ID NO.8 | |
| MTE7071 | First chain | SEQ ID NO.9 | hinge10(IgG1 C220S) |
| | Second chain | SEQ ID NO.10 | |
| MTE7273 | First chain | SEQ ID NO.11 | hinge11(IgG1 C226S) |
| | Second chain | SEQ ID NO.12 | |
| MTE7475 | First chain | SEQ ID NO.13 | hinge12(IgG1 C229S) |
| | Second chain | SEQ ID NO.14 | |
| MTE7677 | First chain | SEQ ID NO.15 | hinge3(IgG1 C220del+C226S) |
| | Second chain | SEQ ID NO.16 | |
| MTE7879 | First chain | SEQ ID NO.17 | hinge4(IgG1 C220del+C229S) |
| | Second chain | SEQ ID NO.18 | |
| MTE8081 | First chain | SEQ ID NO.19 | hinge13(IgG1 C226S+C229S) |
| | Second chain | SEQ ID NO.20 | |
| BEC8283 | First chain | SEQ ID NO.21 | hinge2(IgG1 C220del) |
| | Second chain | SEQ ID NO.22 | |
| BEC2728 | First chain | SEQ ID NO.23 | hinge 1 (IgG 1 WT) |
| | Second chain | SEQ ID NO.24 | |
| BEC2930 | First chain | SEQ ID NO.25 | hinge5(IgG1 C226del) |
| | Second chain | SEQ ID NO.26 | |
| BEC3132 | First chain | SEQ ID NO.27 | hinge6(IgG1 C229del) |
| | Second chain | SEQ ID NO.28 | |
| BEC3334 | First chain | SEQ ID NO.29 | hinge7(IgG1 C220del+C226del) |
| | Second chain | SEQ ID NO.30 | |
| BEC3536 | First chain | SEQ ID NO.31 | hinge8(IgG1C220del+C229del) |
| | Second chain | SEQ ID NO.32 | |
| BEC3738 | First chain | SEQ ID NO.33 | hinge9(IgG1 C226del+C229del) |
| | Second chain | SEQ ID NO.34 | |
| BEC3940 | First chain | SEQ ID NO.35 | hinge10(IgG1 C220S) |
| | Second chain | SEQ ID NO.36 | |
| BEC4142 | First chain | SEQ ID NO.37 | hinge11(IgG1 C226S) |
| | Second chain | SEQ ID NO.38 | |
| BEC4344 | First chain | SEQ ID NO.39 | hinge12(IgG1 C229S) |
| | Second chain | SEQ ID NO.40 | |
| BEC4546 | First chain | SEQ ID NO.41 | hinge13(IgG1 C226S+C229S) |
| | Second chain | SEQ ID NO.42 | |
| BEC9293 | First chain | SEQ ID NO.43 | hinge 14(IgG4 WT) |
| | Second chain | SEQ ID NO.44 | |
| BEC9495 | First chain | SEQ ID NO.45 | hinge 14(IgG4 WT) |
| | Second chain | SEQ ID NO.46 | |
| BEC0607 | First chain | SEQ ID NO.47 | hinge 14(IgG4 WT) |
| | Second chain | SEQ ID NO.48 | |
| BEC9697 | First chain | SEQ ID NO.49 | hingel5(IgG4 C226S) |
| | Second chain | SEQ ID NO.50 | |
| BEC0001 | First chain | SEQ ID NO.51 | hingel5(IgG4 C226S) |
| | Second chain | SEQ ID NO.52 | |
| BEC9899 | First chain | SEQ ID NO.53 | hingel6(IgG4 C229S) |
| | Second chain | SEQ ID NO.54 | |
| BEC0203 | First chain | SEQ ID NO.55 | hingel6(IgG4 C229S) |
| | Second chain | SEQ ID NO.56 | |
| BEC0405 | First chain | SEQ ID NO.57 | hinge 17(IgG4 C226S+C229S) |
| | Second chain | SEQ ID NO.58 | |
| BEC0809 | First chain | SEQ ID NO.59 | hinge18(IgG4 C226del) |
| | Second chain | SEQ ID NO.60 | |
| BEC1011 | First chain | SEQ ID NO.61 | hinge 19(IgG4 C229del) |
| | Second chain | SEQ ID NO.62 | |
| BEC8485 | Second chain | SEQ ID NO.63 | hinge2(IgG1 C220del) |
| | First chain | SEQ ID NO.64 | |
| BEC0910 | First chain | SEQ ID NO.65 | hinge3(C220del+C226S) |
| | Second chain | SEQ ID NO.66 | |
| BEC1112 | First chain | SEQ ID NO.67 | hinge3(IgG1 C220del+C226S) |
| | Second chain | SEQ ID NO.68 | |
| BEC1314 | First chain | SEQ ID NO.69 | hinge4(IgG1 C220del+C229S) |
| | Second chain | SEQ ID NO.70 | |
| BEC1516 | Second chain | SEQ ID NO.71 | hinge4(IgG1 C220del+C29S) |
| | First chain | SEQ ID NO.72 | |
| BEC0102 | First chain | SEQ ID NO.73 | hinge2(IgG1 C220del) |
| | Second chain | SEQ ID NO.74 | |
| BEC0304 | First chain | SEQ ID NO.75 | hinge2(IgG1 C220del) |
| | Second chain | SEQ ID NO.76 | |
| BEC6162 | First chain | SEQ ID NO.77 | hinge2(IgG1 C220del) |
| | Second chain | SEQ ID NO.78 | |
| BEC6364 | First chain | SEQ ID NO.79 | hinge2(IgG1 C220del) |
| | Second chain | SEQ ID NO.80 | |
| hinge1(IgG1 WT) | | SEQ ID NO.81 | EPKSCDKTHTCPPCP |
| hinge2(IgG1 C220del) | | SEQ ID NO.82 | EPKSDKTHTCPPCP |
| hinge3(IgG1 C220del+C226S) | | SEQ ID NO.83 | EPKSDKTHTSPPCP |
| hinge4(IgG1 C220del+C29S) | | SEQ ID NO.84 | EPKSDKTHTCPPSP |
| hinge5(IgG1 C226del) | | SEQ ID NO.85 | EPKSCDKTHTPPCP |
| hinge6(IgG1 C229del) | | SEQ ID NO.86 | EPKSCDKTHTCPPP |
| hinge7(IgG1 C220del+C226del) | | SEQ ID NO.87 | EPKSDKTHTPPCP |
| hinge8(IgG1 C220del+C229del) | | SEQ ID NO.88 | EPKSDKTHTCPPP |
| hinge9(IgG1 C226del+C229del) | | SEQ ID NO.89 | EPKSCDKTHTPPP |
| hinge10(IgG1 C220S) | | SEQ ID NO.90 | EPKSSDKTHTCPPCP |
| hinge11(IgG1 C226S) | | SEQ ID NO.91 | EPKSCDKTHTSPPCP |
| hinge12(IgG1 C229S) | | SEQ ID NO.92 | EPKSCDKTHTCPPSP |
| hinge13(IgG1 C226S+C229S) | | SEQ ID NO.93 | EPKSCDKTHTSPPSP |
| hinge14(IgG4 WT) | | SEQ ID NO.94 | ESKYGPPCPPCP |
| hinge15(IgG4 C226S) | | SEQ ID NO.95 | ESKYGPPSPPCP |
| hinge16(IgG4 C229S) | | SEQ ID NO.96 | ESKYGPPCPPSP |
| hinge17(IgG4 C226S+C229S) | | SEQ ID NO.97 | ESKYGPPSPPSP |
| hinge18(IgG4 C226del) | | SEQ ID NO.98 | ESKYGPPPPCP |
| hinge19(IgG4 C229del) | | SEQ ID NO.99 | ESKYGPPCPPP |
| hinge20(IgG2 WT) | | SEQ ID NO.100 | ERKCCVECPPCP |
| hinge21 | | SEQ ID NO.101 | DKTHTCPPCP |
| PEC3132 | First chain | SEQ ID NO.102 | hinge1(IgG1 WT) |
| | Second chain | SEQ ID NO.103 | hinge21 |
| PEC9903 | First chain | SEQ ID NO.104 | hinge21 |
| | Second chain | SEQ ID NO.105 | hinge1(IgG1 WT) |
| PEC1415 | First chain | SEQ ID NO.106 | hinge21 |
| | Second chain | SEQ ID NO.107 | hinge2(IgG1 C220del) |
| PEC1516 | First chain | SEQ ID NO.107 | hinge2(IgG1 C220del) |
| | Second chain | SEQ ID NO.108 | hinge21 |
| MTE8687 | First chain | SEQ ID NO:109 | hinge2(IgG1 C220del) |
| | Second chain | SEQ ID NO:110 | |
| MTE9091 | First chain | SEQ ID NO:111 | hinge2(IgG1 C220del) |
| | Second chain | SEQ ID NO:112 | |

### Example 2. Expression and Purification of Fusion Antibodies

The eukaryotic expression vector plasmids constructed in Example 1 (see Table 1) were co-transfected into FreeStyle HEK293 cells at a cell density of 2.5 x 10⁶ cells/ml, and cultured for 5-6 days at 125 rpm, 37°C, and 5% CO₂. The cell culture supernatant was collected by centrifugation, and filtered through a 0.22 µm filter. The fusion antibodies were purified using Protein A Resin (Genscript) according to the manufacturer's instructions. The concentration was determined by A280 and BCA (Pierce). The fusion antibodies purified by Protein A resin were further purified and subjected to buffer-exchange using a GE AKTA chromatography system with a Superdex 200 Increase 10/300 GL size exclusion chromatography column in PBS buffer (pH 7.4). The purified samples were stored in PBS buffer (pH 7.4). The composition and purity of the fusion antibodies were assessed by SDS-PAGE under reducing and non-reducing conditions, and the results are shown in Figures 2A-2D. No impurity bands were observed in lanes 2, 5, 8, and 9 under reducing conditions in FIG. 2A and in lanes 2, 5, 6, and 8 in FIG. 2B, indicating high purity, whereas obvious non-target bands appeared at 130-250 kD in the remaining lanes, indicating that mutation or deletion of the first Cys on the hinge can effectively reduce formation of non-target bands.

### Example 3. Preparation of Fusion Antibody Conjugates

In this study, conjugates were prepared based on three toxin molecules, MMAE, Dxd, and Dupa. A certain amount of the fusion antibody of Example 2 were subjected to Cys-based site-specific conjugation. By optimizing the reduction reaction using tris(2-carboxyethyl)phosphine hydrochloride (abbreviated as TCEP, CAS: 51805-45-9), the interchain disulfide bonds of the antibody were opened. A payload with a maleimide linker was added (Payload 1, Deruxtecan, CAS: 1599440-13-7; Payload 2, vc-MMAE, CAS: 646502-53-6), and an alkylation reaction occurred through nucleophilic Michael addition of thiol groups, forming stable thioether bonds and producing relatively homogeneous conjugates. In the reduction reaction, the heterodimeric fusion protein and TCEP were reacted at a molar ratio of 1/20 in a 37°C incubator for 60 minutes. After the reaction, the sample was removed and cooled on ice, and then a pre-cooled linker-containing Payload was added at a molar ratio of 20/1 to the heterodimeric fusion protein, and reacted on ice for 2 hours. TCEP introduced by the reduction reaction and excess unreacted Payload were removed through a 40 kDa Zeba desalting spin column (CAS: 87766), and the conjugated sample was simultaneously exchanged from the previous DPBS buffer system into a suitable buffer system (10 mg/mL sucrose, 20 mg/mL glycine, 1.47 mg/mL glutamic acid, pH 4.0). The composition and purity of the conjugates were detected by SDS-PAGE under reducing and non-reducing conditions. The results (see Figures 3A-3F) showed that the sizes of the fusion antibodies conjugated with Dxd, MMAE, or DUPA under both non-reducing and reducing conditions were all as expected. No single-chain dropouts at 55 kDa or 70 kDa were observed under non-reducing conditions, indicating that the fusion antibodies and their conjugates had intact structures under non-reducing conditions.

### Example 4. Stability Testing

The fusion antibodies of Example 2 and the conjugates of Example 3 were mixed with freshly prepared thermal shift dye and shift buffer (Protein Thermal Shift^{™} Dye Kit, ThermoFisher Scientific, Cat. 4461146) at the ratio recommended by the manufacturer. Thermal scanning was performed using a ViiA^{™} 7 Real-Time PCR System at a heating rate of 0.05°C/s from 25°C to 99°C, or at a heating rate of 1.0 C/min from 20°C to 95°C using a Prometheus NT.48 protein stability analyzer. The thermal melting temperature (Tm) values were calculated using the "Area under curve (AUC)" analysis model in GraphPad Prism 7 software. Atezolizumab (Genscript) was used as a monoclonal antibody control sample. Each set of data was repeated twice to ensure reproducibility of the results.

The results are shown in Table 2: The aggregation temperature (Tagg) value of BEC8283 was relatively high, substantially consistent with that of BEC2728, and higher than the Tagg values of other formats in the table, indicating that deletion of the first Cys on the hinge does not cause a change in protein aggregation temperature, i.e., does not affect protein stability. In addition, the initial melting temperature (Tm-1) value of BEC8283 was relatively high, and substantially consistent with that of BEC2728.

The initial melting temperature (Tm-1) value of MTE6465 was higher than those of other formats in the table (Table 3). The initial melting temperature (Tm-1) values of BEC9495 and BEC0607 were relatively high, and substantially consistent with that of BEC9293 (Table 4). This indicates that changing the first Cys on the hinge has little effect on the thermal stability of the protein structure.

**Table 2 Tm and Tagg values of fusion antibodies**

| Sample | Tagg (°C) | Tm-1 (°C) | Tm-2 (°C) |
|---|---|---|---|
| BEC2728 | 71.3 | 71.1 | 76.5 |
| BEC8283 | 71.1 | 70.8 | 76.7 |
| BEC2930 | 57.7 | 71.1 | 76.6 |
| BEC3132 | 56.4 | 70.5 | 76.7 |
| BEC3334 | 56.6 | 70.8 | 76.7 |
| BEC3536 | 57.7 | 70.5 | 76.6 |
| BEC3738 | 57.9 | 69.4 | 76.7 |
| BEC3940 | 55.8 | 70.7 | 76.6 |
| BEC4142 | 57.4 | 71.1 | 76.6 |
| BEC4344 | 57.2 | 69.7 | 76.4 |
| BEC4546 | 57.0 | 67.3 | 76.3 |

**Table 3 Tm values of fusion antibodies**

| Sample | Tm-1 (°C) | Tm-2 (°C) | Tm-3 (°C) |
|---|---|---|---|
| MTE6263 | 68.83 | 74.39 | -- |
| MTE6465 | 69.03 | 74.19 | -- |
| MTE6667 | 63.28 | 67.64 | 74.19 |
| MTE6869 | 62.68 | 67.25 | 74.19 |
| MTE7071 | 62.68 | 67.84 | 74.59 |
| MTE7273 | 68.64 | 74.59 | -- |
| MTE7475 | 61.69 | 74.19 | -- |
| MTE7677 | 62.88 | 74.59 | -- |
| MTE7879 | 62.49 | 66.65 | 73.79 |
| MTE8081 | 65.46 | 74.39 | -- |

**Table 4 Tm values of fusion antibodies**

| Sample | Tm (°C) |
|---|---|
| BEC9293 | 64.07 |
| BEC9495 | 64.67 |
| BEC9697 | 63.48 |
| BEC9899 | 63.28 |
| BEC0001 | 63.68 |
| BEC0203 | 63.28 |
| BEC0405 | 63.28 |
| BEC0607 | 65.26 |
| BEC0809 | 57.33 |
| BEC1011 | 58.72 |

**Table 5 Tm values of fusion antibodies and conjugates**

| Sample | Tm1 | Tm2 |
|---|---|---|
| Atezolizumab | 60.79°C | 84.98°C |
| BEC-8283 | 62.92°C | 81.11°C |
| BEC-8283-Dxd | 61.58°C | 71.26°C |
| BEC-8283-MMAE | 61.58°C | 79.38°C |
| BEC-8485 | 61.55°C | 80.69°C |
| BEC-8485-Dxd | 61.74°C | 79.34°C |
| BEC-8485-MMAE | 62.52°C | 80.11°C |
| BEC-1314 | 61.26°C | 83.85°C |
| BEC-1314-MMAE | 67.99°C | 83.34°C |

### Example 5. Mass Spectrometry Analysis

The fusion antibodies and their conjugates in Example 3 were incubated with PNGase F (NEB) overnight at 37°C at a concentration of 1 mg/ml. The deglycosylated samples were reduced by addition of 10 mM DTT. The reduced samples were injected into a 300SB-C8, 2.1 x 50 mm column of an HPLC-Q-TOF-MS (Agilent, USA) for MS analysis, and DAR values were calculated using the "Area Under Curve (AUC)" analysis model in GraphPad Prism 7 software. As shown in Figure 4, the molecular weights of the fusion antibodies and their conjugates were essentially consistent with the theoretical predicted values. Both chains of the Dxd-conjugated fusion antibody carried a specific number of small molecule drugs, and the number corresponded to the number of reduced Cys in the hinge region, with a fixed DAR value.

### Example 6. Antigen Binding Activity Assay

Target antigens (100 ng/100 µl/well) were coated onto a 96-well ELISA plate and incubated overnight at 4°C. After blocking at room temperature for 1 h with PBST containing 3% skim milk powder (0.5% Tween-20 in PBS) for 1 hour at room temperature, the plate was washed with PBST. The fusion antibody and corresponding conjugate samples were diluted to 50 nM with blocking buffer and, using this as the starting concentration, subjected to 5-fold serial dilution for a total of 8 dilution gradients. 100 µl of the diluted fusion antibodies and corresponding control samples were added to each well and incubated for 1 hour at room temperature. After washing three times with PBST, 100 µl of HRP-labeled goat anti-human Fc monoclonal antibody diluted 1:500 according to the manufacturer's instructions, was added to each well and incubated at room temperature for 1 hour, followed by washing five times with PBST. 100 µl of freshly prepared TMB substrate (BioLegend, Cat. 421101) was added to the washed ELISA wells and incubated at room temperature protected from light for 10 minutes. Then, 100 µl of 1M H₂SO₄ was added to each well, and incubated for 2 minutes for color development, and readings were taken at 450 nm using a microplate reader. EC50 values were calculated using the "log(agonist) vs. response -- Variable slope (four parameters)" analysis model of GraphPad Prism 7 software. Each set of data was repeated twice to ensure reproducibility. As shown in Figure 5, the binding abilities of fusion antibodies BEC8283 and BEC8485 to the target antigens cMet and EGFR were similar to those of the controls.

### Example 7. Phosphorylation Assay by Western Blot

1 x 10⁶ cells were seeded in six-well plates and incubated at 37°C for 12 hours. The medium was then removed and replaced with serum-free medium for starvation for 24 hours. Fusion antibody samples, control samples, EGF (50 ng/ml), or HGF (100 ng/ml) were respectively added to the starved cells, followed by incubation at 37°C for 15 minutes. The incubated cell samples were washed with pre-cooled DPBS, cell lysis buffer was added, and the cells were lysed on ice for 1 hour. The lysate supernatant was collected by centrifugation, and quantified using a BCA kit. Equal amounts of cell lysate samples were mixed with loading dye containing DTT, heated at 95°C for 10 minutes, and then subjected to SDS-PAGE. The SDS-PAGE gel were transferred to a PVDF membrane at low temperature using a transfer apparatus. After the transferred PVDF membrane was blocked at room temperature for 1 hour, the corresponding primary and secondary antibodies were incubated. After incubation with the secondary antibody was completed, the membrane was thoroughly washed, substrate was added for color development, and images were taken.

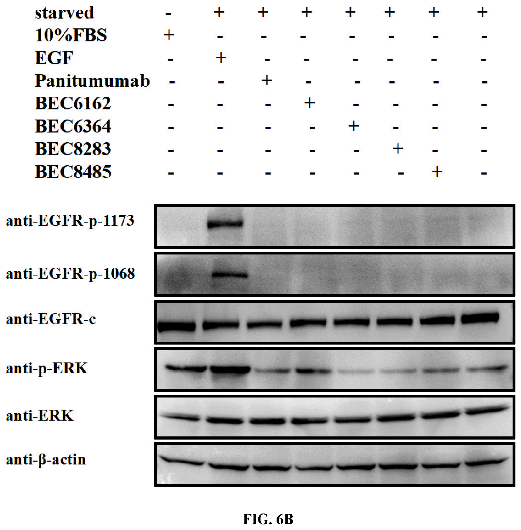

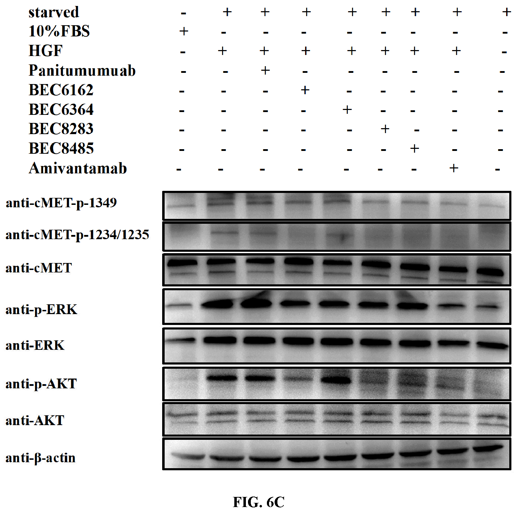

The results are shown in Figures 6A-6D. In A549 cells, under EGF stimulation, the heterodimeric fusion protein completely inhibited EGFR phosphorylation and partially inhibited the phosphorylation of the proliferation signaling protein ERK (Figure 6A). In the absence of EGF stimulation, the heterodimeric fusion protein still exhibited a good inhibitory effect on ERK phosphorylation (Figure 6B). Regardless of the presence or absence of HGF induction, the heterodimeric fusion protein showed good inhibitory effects on phosphorylation of ERK and AKT. When HGF induction was added, the heterodimeric fusion protein further inhibited the phosphorylation of cMET-1349 and cMET-1234/1235 (Figures 6C-6D).

### Example 8. Pharmacokinetic Evaluation in Mice

6-8 week old C57BL6J mice were administered via tail vein injection. The fusion antibody was administered at a single high dose of 10 mg/kg. The fusion antibody conjugates were administered at three doses: 8 mg/kg, 4 mg/kg, and 1 mg/kg. Blood was collected from the orbital sinus at different time points. The concentration of intact fusion antibody and its conjugates in serum at each time point was measured by ELISA, and the data were processed using GraphPad Prism. The results, shown in the table, indicate that the fusion antibody conjugates exhibited pharmacokinetic profiles similar to that of the fusion antibody.

**Table 6 In vivo half-lives of fusion antibodies and conjugates thereof**

| | **t1/2 for BsAb** | **t1/2 for BsAb-ADC** |
|---|---|---|
| BEC-8283(10mg/kg,IV) | 250.76h | -- |
| BEC-8283-MMAE(8mg/kg, IV) | 103.76h | 65.42h |
| BEC-8283-MMAE(4mg/kg, IV) | 103.76h | 68.39h |
| BEC-8283-MMAE(1mg/kg, IV) | 111.45h | 64.02h |

### Example 9. Pharmacodynamic Evaluation in Mice

6-8 week old nude mice were subcutaneously inoculated with tumor cells. On day 0, 5 x 10⁶ NCI-H1975 cells were inoculated, and on day 8, when the tumor size reached approximately 100-200 mm³, a single tail vein administration was performed. The fusion antibody conjugates were administered at doses of 5, 2.5, and 1 mg/kg, and the corresponding fusion antibody was administered at doses of 5, 2.5, and 1 mg/kg. Tumor sizes were monitored three times per week. The results are shown in Figure 7. The fusion antibody BEC8283 exhibited good tumor inhibitory effects in NCI-H1975 tumor-bearing mice in a dose-dependent manner. The BEC8283 conjugates exhibited a better tumor inhibitory effect than the fusion antibody.

## Claims

1. A fusion antibody comprising:
(a) a first polypeptide chain comprising, from N-terminus to C-terminus, a heavy chain variable region (VH), a first heavy chain constant region (CH1), a hinge region (hinge) and a first Fc;
(b) a second polypeptide chain comprising, from N-terminus to C-terminus, a light chain variable region (VL), a light chain constant region (CL), a hinge region (hinge) and a second Fc;
wherein the cysteine (Cys) at position 214 of CL of the second polypeptide chain is deleted (C214del, EU numbering, hereinafter EU numbering is adopted); and said VH and said VL constitute a first antigen-binding domain.

2. The fusion antibody according to claim 1, wherein the hinges of the first polypeptide chain and the second polypeptide chain are different.

3. The fusion antibody according to claim 2, wherein the hinge of the first polypeptide chain and the second polypeptide chain lacks Cys at position 220(C220del).

4. The fusion antibody according to claim 3, wherein the first polypeptide chain has a hinge as shown in EPKSDKTHTCPPCP (SEQ ID NO: 82), and the second polypeptide chain has a hinge as shown in DKTHTCPPCP (SEQ ID NO: 101).

5. The fusion antibody according to claim 1, wherein the hinges of the first polypeptide chain and the second polypeptide chain are identical.

6. The fusion antibody according to claim 5, wherein the hinge of the first polypeptide chain and the second polypeptide chain is selected from IgG1, said hinge is EPKSCDKTHTCPPCP (E216-P230) (SEQ ID NO: 81), and one or more Cys on said hinge are deleted and/or mutated to serine (Ser), threonine (Thr) or glycine (Gly).

7. The fusion antibody according to claim 6, wherein the first Cys on the hinge of the first polypeptide chain and the second polypeptide chain is deleted (C220del) or mutated to Ser (C220S).

8. The fusion antibody according to claim 7, wherein the second Cys on the hinge of the first polypeptide chain and the second polypeptide chain is deleted (C226del) or mutated to Ser (C226S).

9. The fusion antibody according to claim 7, wherein the third Cys on the hinge of the first polypeptide chain and the second polypeptide chain is deleted (C229del) or mutated to Ser (C229S).

10. The fusion antibody according to any one of claims 1-9, wherein,
the first Fc and the second Fc are derived from IgG1, IgG2, IgG3 or IgG4;
the first Fc and the second Fc comprise amino acid substitutions that promote heterodimerization of the first Fc and the second Fc.

11. The fusion antibody according to claim 10, wherein:
one of the first Fc and the second Fc comprises amino acid substitution T366W, and the other comprises amino acid substitutions T366S, L368A and Y407V;
one of the first Fc and the second Fc comprises amino acid substitutions T366W and S354C, and the other comprises amino acid substitutions T366S, L368A, Y407V and Y349C; or
one of the first Fc and the second Fc comprises amino acid substitutions E356K and R409K, and the other comprises amino acid substitutions R409K and K439E.

12. The fusion antibody according to any one of claims 1-10, wherein the N-terminus of one of the first polypeptide chain and the second polypeptide chain is further fused with an antibody heavy chain variable region (VH2), and the N-terminus of the other is further fused with an antibody light chain variable region (VL2), wherein VH2 and VL2 form a second antigen-binding domain.

13. The fusion antibody according to any one of claims 1-12, wherein the fusion antibody has a first polypeptide chain and a second polypeptide chain composed of the following amino acid sequences: SEQ ID NO:21 and SEQ ID NO:22; SEQ ID NO:3 and SEQ ID NO:4; SEQ ID NO:9 and SEQ ID NO:10; SEQ ID NO:3 and SEQ ID NO:4; SEQ ID NO:15 and SEQ ID NO:16; SEQ ID NO:17 and SEQ ID NO:18; SEQ ID NO:29 and SEQ ID NO:30; SEQ ID NO:31 and SEQ ID NO:32; SEQ ID NO:35 and SEQ ID NO:36; SEQ ID NO:63 and SEQ ID NO:64; SEQ ID NO:65 and SEQ ID NO:66; SEQ ID NO:67 and SEQ ID NO:68; SEQ ID NO:69 and SEQ ID NO:70; SEQ ID NO:71 and SEQ ID NO:72; SEQ ID NO:77 and SEQ ID NO:78; SEQ ID NO:79 and SEQ ID NO:80; SEQ ID NO:106 and SEQ ID NO:107; SEQ ID NO:107 and SEQ ID NO:108; SEQ ID NO:109 and SEQ ID NO:110; SEQ ID NO:111 and SEQ ID NO:112.

14. A conjugate comprising the fusion antibody of any one of claims 1-13, and a cytotoxic drug conjugated to Cys on the hinge of the fusion antibody.

15. A polynucleotide encoding the fusion antibody of any one of claims 1-13.

16. A vector comprising the polynucleotide of claim 15.

17. A host cell comprising the vector of claim 16.

18. A pharmaceutical composition comprising the fusion antibody of any one of claims 1-13 or the conjugate of claim 14.
